# EUROPEAN PATENT SPECIFICATION

(11) **EP 3 508 311 B1**
(45) Date of publication and mention of the grant of the patent: **09.03.2022**
(21) Application number: 17845011.0
(22) Date of filing: 29.06.2017
(51) Int. Cl.: B25J 11/00, A61B 34/30, A61B 34/00

(54) **TRANSMISSION MECHANISM AND SURGICAL INSTRUMENT**
ÜBERTRAGUNGSMECHANISMUS UND CHIRURGISCHES INSTRUMENT
MÉCANISME DE TRANSMISSION ET INSTRUMENT CHIRURGICAL

(30) Priority: 31.08.2016 CN 201610791555
(43) Date of publication of application: 10.07.2019
(73) Proprietor: Shanghai Microport Medbot (Group) Co., Ltd., Shanghai 201203 (CN)
(72) Inventor: HE, Chao, Shanghai 201203 (CN); HE, Yuyuan, Shanghai 201203 (CN); WANG, Changchun, Shanghai 201203 (CN); YUAN, Shuai, Shanghai 201203 (CN)
(74) Representative: Patentship Patentanwaltsgesellschaft mbH
(86) International application number: PCT/CN2017/090720
(87) International publication number: WO 2018/040706

(56) References cited:
- CN-A- 101 732 093
- CN-A- 102 119 872
- CN-A- 102 499 757
- CN-A- 105 025 829
- CN-A- 105 286 999
- CN-A- 106 308 933
- DE-A1-102013 225 117
- US-A1- 2002 120 363

## Description

### TECHNICAL FIELD

The present invention relates to the technical field of medical instruments, and in particular, to a transmission mechanism applicable to minimally invasive surgical robots.

### BACKGROUND

Compared to conventional open surgical operations, the minimally invasive surgery has obvious advantages in terms of therapeutic effect, pain relief, recovery cycle, and medical cost, etc., and thus is widely used in the surgical procedure.

In recent years, with the development of the minimally invasive surgery technology, surgical robots become more and more popular, and are used in surgeries of increasingly wide scope, increasingly complicated patient's condition with increasingly improved operation precision and intelligential degree. At present, the representative robots are the Da Vinci surgical robot system developed by Intuitive Surgical Company of the United States and the ZEUS surgical robot system developed by Computer Motion Company, which have been successfully used in surgical operation. However, both types of such surgical robot systems have the disadvantages of complicated mechanism, large size, and high price, etc.

In particular, it is found that the performance of the entire surgical robot is directly affected and determined by the property of the surgical instruments, and the transmission mechanism (disposed in an instrument set) for driving the movement of the surgical instruments is a key factor that restricts the performance of the surgical instruments. However, the transmission mechanism disclosed in US Patent No. 9,078,684 B2 uses six wire ropes to drive three joints located at the end of the surgical instrument to achieve three degrees of freedom movement, which causes the defects of a complicated transmission structure and a large space occupation. Therefore, it is necessary to develop a transmission mechanism with a simple transmission structure and a small space occupation to drive an instrument terminal to achieve three degrees of freedom movement.

Chinese patent application CN 105 286 999 A discloses a minimally invasive surgery instrument with a tail end self-rotation function.

Chinese patent application CN 102 119 872 A discloses a compact quick-change mechanism of a robot for minimally invasive surgery, which comprises a quick-change box and a quick-change base.

### SUMMARY

The invention is described in the independent claim. Preferred embodiments of the invention are illustrated in the dependent claims.

It is an object of the present invention to provide a transmission mechanism and a surgical instrument including same. Compared with the transmission mechanism of the prior art, the present transmission mechanism has a simple structure, a low control difficulty and a small space occupation, and is also able to drive an instrument terminal located at the end of the surgical instrument to achieve three degrees of freedom movement.

To achieve the foregoing object and other related objects, the present invention provides a transmission mechanism disposed on a surgical instrument and configured to drive an instrument terminal located at the end of the surgical instrument to move, the transmission mechanism comprising:
driving shafts comprising a first driving shaft, a second driving shaft and a third driving shaft which are arranged in parallel;
tractors comprising a first tractor, a second tractor, a third tractor and a fourth tractor; and
a guide mechanism;
wherein the first tractor and the second tractor are wound around the first driving shaft in opposite directions, partially fixedly connected to the first driving shaft, and fixedly connected to the instrument terminal after changing directions via the guide mechanism; and the third tractor and the fourth tractor are wound around the third driving shaft in opposite directions, partially fixedly connected to the third driving shaft, and fixedly connected to the instrument terminal after changing directions via the guide mechanism;
wherein the guide mechanism is configured such that when the second driving shaft rotates, the movement direction of portions of the first tractor and the second tractor located between the guide mechanism and the instrument terminal is opposite to the movement direction of portions of the third tractor and the fourth tractor located between the guide mechanism and the instrument terminal.

Preferably, the guide mechanism comprises a first guide mechanism and a second guide mechanism, with axes of the first guide mechanism and the second guide mechanism being parallel to axes of the driving shafts;
wherein the guide mechanism is located in the first space defined by a first plane formed by axes of the first driving shaft and the second driving shaft, and a second plane formed by axes of the second driving shaft and the third driving shaft; the first guide mechanism is farther from the first driving shaft than the second guide mechanism, and the second guide mechanism is farther from the third driving shaft than the first guide mechanism;
wherein the first tractor and the second tractor, wound around the first driving shaft in opposite directions and partially fixedly connected to the first driving shaft, are fixedly connected to the instrument terminal after changing directions by passing through the same side of the first guide mechanism; and the third tractor and the fourth tractor wound around the third driving shaft in opposite directions and partially fixedly connected to the third driving shaft, are fixedly connected to the instrument terminal after changing directions by passing through the same side of the second guide mechanism .

Preferably, the transmission mechanism further comprises:
connectors comprising a first connector and a second connector;
wherein the first connector and the second connector are not intersected, and are each perpendicular to the axis of the second driving shaft; one end of the first connector is fixedly connected to the second driving shaft, with the other end of the first connector being fixedly connected to the first guide mechanism; and one end of the second connector is fixedly connected to the second driving shaft, with the other end of the second connector being fixedly connected to the second guide mechanism.

Preferably, the transmission mechanism further comprises:
an output guide mechanism comprising a first output guide mechanism and a second output guide mechanism;
wherein the output guide mechanism is located in a second space defined by a third plane formed by axes of the first driving shaft and the third driving shaft, a fourth plane formed by axes of the second driving shaft and the first guide mechanism, and a fifth plane formed by axes of the second driving shaft and the second guide mechanism; the axes of the first output guide mechanism and the second output guide mechanism are perpendicular to the axis of the second driving shaft, and the axes of the first output guide mechanism and the second output guide mechanism are not intersected;
wherein the first tractor and the second tractor, after changing directions by passing through the same side of the first guide mechanism, are fixedly connected to the instrument terminal by further passing through the first output guide mechanism; and the third tractor and the fourth tractor, after changing directions by passing through the same side of the second guide mechanism, are fixedly connected to the instrument terminal by further passing through the second output guide mechanism.

Preferably, the guide mechanism further comprises a third mechanism and a fourth guide mechanism which are sleeved on the second driving shaft, and the third guide mechanism does not coincide with the fourth guide mechanism in the axial direction of the second driving shaft;
the first tractor and the second tractor, after being wound around the first driving shaft and changing directions by passing through one side of both the first guide mechanism and the third guide mechanism , are fixedly connected to the instrument terminal by further passing through the first output guide mechanism; and the third tractor and the fourth tractor, after being wound around the third driving shaft and changing directions by passing through one side of both the second guide mechanism and the fourth guide mechanism, are fixedly connected to the instrument terminal by further passing through the second output guide mechanism.

Preferably, the guide mechanism further comprises a third mechanism and a fourth guide mechanism; the fourth guide mechanism is farther from the first driving shaft than the third guide mechanism, and the third guide mechanism is farther from the third driving shaft than the fourth guide mechanism;
Wherein the first tractor and the second tractor, after being wound around the first driving shaft and changing directions by passing through one side of both the first guide mechanism and the third guide mechanism , are fixedly connected to the instrument terminal by further passing through the first output guide mechanism; and the third tractor and the fourth tractor, after being wound around the third driving shaft and changing directions by passing through one side of both the second guide mechanism and the fourth guide mechanism, are fixedly connected to the instrument terminal by further passing through the second output guide mechanism.

Preferably, the transmission mechanism further comprises:
connectors comprising a third connector and a fourth connector, wherein the third connector and the fourth connector are not intersected, and are each perpendicular to the axis of the second driving shaft; one end of the third connector is fixedly connected to the second driving shaft, with the other end of the third connector being fixedly connected to the third guide mechanism; and one end of the fourth connector is fixedly connected to the second driving shaft, with the other end of the fourth connector being fixedly connected to the fourth guide mechanism.

Preferably, the first tractor and the second tractor pass through the same side of the first guide mechanism and the third guide mechanism, and the third tractor and the fourth tractor pass through the same side of the second guide mechanism and the fourth guide mechanism.

Preferably, the distance between the axis of the second driving shaft and the axis of the first driving shaft is equal to the distance between the axis of the second driving shaft and the axis of the third driving shaft.

Preferably, the first tractor and the second tractor pass through the first guide mechanism from one side of the first guide mechanism away from the second driving shaft, and the third tractor and the fourth tractor pass through the second guide mechanism from one side of the second guide mechanism away from the second driving shaft.

Preferably, the output guide mechanism is located on a mid-vertical plane of the third plane, or the first output guide mechanism and the second output guide mechanism are symmetrical with respect to the mid-vertical plane.

Preferably, the first tractor comprises a first end, a first body extending from the first end, and a second end connected to the first body; the first end is wound around the first driving shaft and is partially fixedly connected to the first driving shaft; the first body passes through one side of the guide mechanism, and extends to the second end after the direction thereof is changed by the first output guide mechanism; and the second end is fixedly connected to the instrument terminal;
the second tractor comprises a third end, a second body extending from the third end, and a fourth end connected to the second body; the third end is wound around the first driving shaft in a direction opposite to the first end, and is partially fixedly connected to the first driving shaft; the second body passes through one side of the guide mechanism, and extends to the fourth end after the direction thereof is changed by the first output guide mechanism; and the fourth end is fixedly connected to the instrument terminal.

Preferably, the third tractor comprises a fifth end, a third body extending from the fifth end, and a sixth end connected to the third body; the fifth end is wound around the third driving shaft and is partially fixedly connected to the third driving shaft; the third body passes through one side of the guide mechanism, and extends to the sixth end after the direction thereof is changed by the second output guide mechanism; and the sixth end is fixedly connected to the instrument terminal;
the fourth tractor comprises a seventh end, a fourth body extending from the seventh end, and an eighth end connected to the fourth body; the seventh end is wound around the third driving shaft in a direction opposite to the fifth end, and is partially fixedly connected to the third driving shaft; the fourth body passes through one side of the guide mechanism, and extends to the eighth end after the direction thereof is changed by the second output guide mechanism; eighth end is fixedly connected to the instrument terminal; and the third body and the fourth body are always located on a same side of the first body and the second body.

Preferably, the first driving shaft and/or the third driving shaft further comprises a rotator; the rotator is fixedly connected to the corresponding driving shaft; the rotator is provided with a hole; and the tractor is wound around the rotator of the corresponding driving shaft and is fixedly connected to the hole of the rotator.

To achieve the foregoing object and other related objects, the present invention provides a surgical instrument, comprising the transmission mechanism according to any one of the embodiments, wherein the transmission mechanism is configured to drive an instrument terminal located at the end of the surgical instrument to move.

In conclusion, the transmission mechanism of the present invention is mainly used on a surgical instrument and configured to drive an instrument terminal of the surgical instrument to move. It only comprises four tractors, three driving shafts and a guide mechanism, thus enabling usage of a less number of parts and a low usage cost. The transmission mechanism of the present invention drives the instrument terminal to perform three degrees of freedom movement merely by driving corresponding four tractors to move through the rotation of the three driving shafts, thus enabling a simple driving structure and a convenient driving method. Furthermore, compared with the existing transmission mechanism having six wire ropes, the transmission mechanism of the present invention, using just four tractors to achieve three degrees of freedom movement, reduces the number of wire ropes and corresponding components, optimizes s the dimension, and simplifies the driving control, thereby resulting in a smaller size of the surgical instrument which results in a smaller minimally invasive wound as well as an excellent operation flexibility due to the more flexible movement of the surgical instruments in the narrow space of the human body. Moreover, the transmission mechanism driven by the tractors has a light overall weight and a better stability and reliability.

### BRIEF DESCRIPTION OF THE DRAWINGS

FIG. 1 is an axial projection view of a transmission mechanism according to Embodiment 1 of the present invention;
FIG. 2 is an axial projection view of a transmission mechanism according to Embodiment 2 of the present invention;
FIG. 3 is a perspective view of the transmission mechanism shown in FIG. 2;
FIG. 4 is a partial schematic view of the transmission mechanism shown in FIG. 3;
FIG. 5 is an axial projection view of a transmission mechanism according to Embodiment 3 of the present invention;
FIG. 6 is a perspective view of a surgical instrument according to one embodiment of the present invention; and
FIG. 7 is a perspective view of a transmission mechanism in an instrument set according to one embodiment of the present invention.

Descriptions of reference numerals in the drawings are as follows:
1-first driving shaft, 2-second driving shaft, 3-third driving shaft, 4-first tractor, 5-second tractor, 6-third tractor, 7-fourth tractor, 8-first guide mechanism, 9-second guide mechanism, 10-first connector, 11-second connector, 12-first output guide mechanism, 13-second output guide mechanism, 14-first rotator, 15-second rotator, 16-third guide mechanism, 17-fourth guide mechanism, 18-third connector, 19-fourth connector, 20-instrument terminal, 21-instrument set, 22-tube, 41-first body, 51-second body, 61-third body, and 71-fourth body.

### DETAILED DESCRIPTION

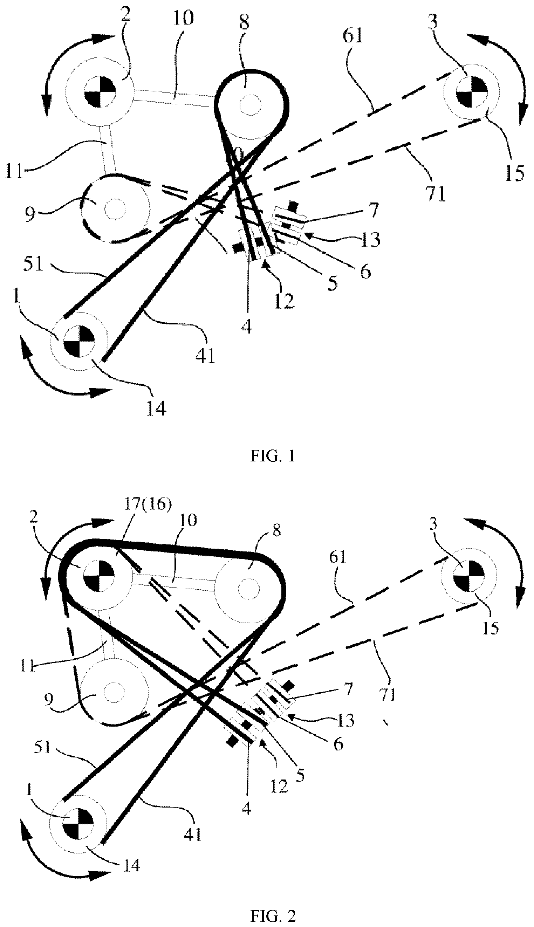

To make the objects, advantages and features of the present invention more clear, the transmission mechanism and the surgical instrument proposed in the present invention will be described in greater detail below with reference to FIGs. 1-7. It should be noted that the accompanying drawings are presented in a very simplified form and not necessarily presented to scale, with the only intention to facilitate convenience and clarity in explaining the object of the present invention.

### <Embodiment 1>

FIG. 1 is an axial projection view of a transmission mechanism according to Embodiment 1 of the present invention. As shown in FIG. 1, the transmission mechanism is disposed on a surgical instrument and configured to drive an instrument terminal located at the end of the surgical instrument to move, and comprises driving shafts, a guide mechanism, and tractors. The driving shafts comprise a first driving shaft 1, a second driving shaft 2 and a third driving shaft 3, which are arranged in parallel. The tractors comprise a first tractor 4, a second tractor 5, a third tractor 6 and a fourth tractor 7.

The first tractor 4 is wound around the first driving shaft 1, partially fixedly connected to the first driving shaft 1, and is fixedly connected to the instrument terminal after changing its direction by passing through one side of the guide mechanism. The second tractor 5 is wound around the first driving shaft 1 in a direction opposite to the first tractor 4, is partially fixedly connected to the first driving shaft 1, and is fixedly connected to the instrument terminal after changing its direction by passing through one side of the guide mechanism. In this way, when the first driving shaft 1 rotates, the first tractor 4 and the second tractor 5 move in opposite directions to achieve a back and forth movement (that is, a tractor moves away from the instrument terminal, and the other tractor moves close to the instrument terminal), so as to drive the instrument terminal to achieve a first degree of freedom movement.

The third tractor 6 is wound around the third driving shaft 3, partially fixedly connected to the third driving shaft 3, and is fixedly connected to the instrument terminal after changing its direction by passing through one side of the guide mechanism. The fourth tractor 7 is wound around the third driving shaft 3 in a direction opposite to the third tractor 6, is partially fixedly connected to the third driving shaft 3, and is fixedly connected to the instrument terminal after changing its direction by passing through one side of the guide mechanism. In this way, when the third driving shaft 3 rotates, the third tractor 6 and the fourth tractor 7 move in opposite directions to achieve a back and forth movement, so as to drive the instrument terminal to achieve a second degree of freedom movement.

According to the invention, the guide mechanism is configured such that when the second driving shaft 2 rotates, the movement direction of the portions of the first tractor 4 and the second tractor 5 located between the guide mechanism and the instrument terminal is opposite to the movement direction of the portions of the third tractor 6 and the fourth tractor 7 located between the guide mechanism and the instrument terminal.

When the first driving shaft 1 rotates, the first tractor 4 and the second tractor 5 move in opposite directions (that is, a tractor moves away from the instrument terminal, and the other tractor moves close to the instrument terminal) to drive the instrument terminal to achieve the first degree of freedom movement. When the third driving shaft 3 rotates, the third tractor 6 and the fourth tractor 7 move in opposite directions to drive the instrument terminal to achieve the second degree of freedom movement. Then, when the second driving shaft 2 rotates, the first tractor 4 and the second tractor 5 move in opposite directions to the third tractor 6 and the fourth tractor 7 to drive the instrument terminal to achieve a third degree of freedom movement. In this way, the transmission mechanism in the present application is able to drive the instrument terminal to achieve movement of three degrees of freedom. For example, the first degree of freedom and the second degree of freedom each may be a degree of freedom of the instrument terminal rotating about a certain axis, and the third degree of freedom may be a degree of freedom of the instrument terminal moving along a certain direction. This can be specifically determined according to actual conditions, and the present invention does not make any limitation.

In this embodiment, the guide mechanism comprises a first guide mechanism 8 and second guide mechanism 9. The axes of the first driving shaft 1, the second driving shaft 2, the third driving shaft 3, the first guide mechanism 8 and the second guide mechanism 9 are parallel to each other. The guide mechanisms are located in a first space. The first space is defined by a first plane formed by the axes of the first driving shaft 1 and the second driving shaft 2 and a second plane formed by the axes of the second driving shaft 2 and the third driving shaft 3; and the first guide mechanism 8 is farther from the first driving shaft 1 than the second guide mechanism 9, and the second guide mechanism 9 is farther from the third driving shaft 3 than the first guide mechanism 8.

The first tractor 4, wound around and partially fixedly connected to the first driving shaft 1, is fixedly connected to the instrument terminal after changing the direction by passing through one side of the first guide mechanism 8. The second tractor 5, wound around the first driving shaft 1 in a direction opposite to the first tractor 4 and partially fixedly connected to the first driving shaft 1, is fixedly connected to the instrument terminal after changing the direction by passing through one side of the first guide mechanism 8. The third tractor 6, wound around and partially fixedly connected to the third driving shaft 3, is fixedly connected to the instrument terminal after changing the direction by passing through one side of the second guide mechanism 9. The fourth tractor 7, wound around the third driving shaft 3 in a direction opposite to the third tractor 6 and partially fixedly connected to the third driving shaft 3, is fixedly connected to the instrument terminal after changing the direction by passing through one side of the second guide mechanism 9. In FIG. 1, the first tractor 4 and the second tractor 5 pass through the same side of the first guide mechanism 8, and the third tractor 6 and the fourth tractor 7 pass through the same side of the second guide mechanism 9. Further, the first tractor 4 and the second tractor 5 pass through the first guide mechanism 8 from the side of the first guide mechanism 8 away from the second driving shaft 2, and the third tractor 6 and the fourth tractor 7 pass through the second guide mechanism 9 from the side of the second guide mechanism 9 away from the second driving shaft 2.

In this embodiment, the transmission mechanism further comprises connectors, i.e., a first connector 10 and a second connector 11. One end of the first connector 10 is fixedly connected to the second driving shaft 2, and the other end of the first connector 10 is fixedly connected to the first guide mechanism 8. The first guide mechanism 8 is preferably rotatable. One end of the second connector 11 is fixedly connected to the second driving shaft 2, and the other end of second connector 11 is fixedly connected to the second guide mechanism 9. The second guide mechanism 9 is preferably rotatable. The first connector 10 and the second connector 11 are not intersected, and are each vertically intersected with the axis of the second driving shaft 2. That is, the intersection point of the first connector 10 and the second driving shaft 2 and the intersection point of the second connector 11 and the second driving shaft 2 are located at different heights of the second driving shaft 2.

Further, the transmission mechanism further comprises output guide mechanism, i.e., a first output guide mechanism 12 and a second output guide mechanism 13. The output guide mechanism (for example the first output guide mechanism 12 and the second output guide mechanism 13) are located in a second space. The second space is defined by a third plane formed by the axes of the first driving shaft 1 and the third driving shaft 3, a fourth plane formed by the axes of the second driving shaft 2 and the first guide mechanism 8, and a fifth plane formed by the axes of the second driving shaft 2 and the second guide mechanism 9. The axes of the first output guide mechanism 12 and the second output guide mechanism 13 are each perpendicular to the axis of the second driving shaft 2, and the axes of the first output guide mechanism 12 and the second output guide mechanism 13 are not intersected.

In the present application, the features "the axes of the first output guide mechanism 12 and the second output guide mechanism 13 are each perpendicular to the axis of the second driving shaft 2, and the axes of the first output guide mechanism 12 and the second output guide mechanism 13 are not intersected" should be understood as that any plane perpendicular to the axis of the second driving shaft 2 passes through at most one of the axes of the first output guide mechanism 12 and the second output guide mechanism 13.

In the present application, the first space was understood as an enclosed space defined by the first plane, the second plane and the third plane preferably, and correspondingly, the axis of the second driving shaft 2 is located in the first space. The second space was understood as a semi-open space defined by the third plane, the fourth plane and the fifth plane, and correspondingly, the axis of the second driving shaft 2 is not located in the second space.

The first tractor 4, after being wound around the first driving shaft 1 and partially fixedly connected to the first driving shaft 1 and changing the direction by passing through one side of the first guide mechanism 8, is fixedly connected to the instrument terminal by further changing direction by passing through the first output guide mechanism 12. The second tractor 5, after being wound around the first driving shaft 1 in a direction opposite to the first tractor 4 and partially fixedly connected to the first driving shaft 1 and changing the direction by passing through one side of the first guide mechanism 8, is fixedly connected to the instrument terminal by further changing direction by passing through the first output guide mechanism 12.

The third tractor 6, after being wound around the third driving shaft 3 and partially fixedly connected to the third driving shaft 3 and changing direction by passing through one side of the second guide mechanism 9, is fixedly connected to the instrument terminal by further changing the direction by passing through the second output guide mechanism 13. The fourth tractor 7, after being wound around the third driving shaft 3 in a direction opposite to the third tractor 6 and partially fixedly connected to the third driving shaft 3 and changing direction by passing through one side of the second guide mechanism 9, is fixedly connected to the instrument terminal by further changing the direction by passing through the second output guide mechanism 13.

In a preferred embodiment, the distance between the axis of the second driving shaft 2 and the axis of the first driving shaft 1 is equal to the distance between the axis of the second driving shaft 2 and the axis of the third driving shaft 3, that is, the vertical distances of the second driving shaft 2 to each of the first driving shaft 1 and the third driving shaft 3 are equal to facilitate optimizing the dimension of the transmission mechanism.

In another preferred embodiment, the output guide mechanism (for example the first output guide mechanism 12 and the second output guide mechanism 13) is located on a mid-vertical plane of the plane (i.e., the above third plane) formed by the axis of the first driving shaft 1 and the axis of the third driving shaft 3. The "mid-vertical plane" refers to a plane perpendicular to the plane where the axis of the first driving shaft 1 and the axis of the third driving shaft 3 are located, and the distances of any point on the mid-vertical plane to each of the axis of the first driving shaft 1 and the axis of the three driving shafts 3 are equal. Alternatively, the first output guide mechanism 12 and the second output guide mechanism 13 are symmetrically disposed with respect to the mid-vertical plane.

As shown in FIG. 1, when the second driving shaft 2 rotates within a certain angle range, the first connector 10 and the second connector 11 rotate together with the second driving shaft 2, and at the same time, the first guide mechanism 8 and the second guide mechanism 9 connected to the two connectors are also rotated (revolved) about the second driving shaft 2. During the rotation of the first guide mechanism 8, the length of the portions of the first tractor 4between the first guide mechanism 8 and the first driving shaft 1 and the length of the portions of the second tractor 5 between the first guide mechanism 8 and the first driving shaft 1 changes accordingly. Since the length of each of the first tractor 4 and the second tractor 5 is constant, the first tractor 4 and the second tractor 5 generate a back and forth movement. Similarly, the third tractor 6 and the fourth tractor 7 also generate a back and forth movement together. For the specific position of each component shown in FIG. 1, when the second driving shaft 2 rotates counterclockwise within a first angle range, the length of the portions of the first tractor 4 between the first guide mechanism 8 and the first driving shaft 1 and the length of the portions of the second tractor 5 between the first guide mechanism 8 and the first driving shaft 1 become longer, meanwhile the length of the portions of the third tractor 6 between the second guide mechanism 9 and the third driving shaft 3, and the length of the portions of the fourth tractor 7 between the second guide mechanism 9 and the third driving shaft 3 become shorter. Therefore the distal ends of the first tractor 4 and the second tractor 5 will move away from the transmission mechanism, and the distal ends of the third tractor 6 and the fourth tractor 7 will move close to the transmission mechanism. Otherwise, when the second driving shaft 2 rotates clockwise within a second angle range, the distal ends of the third tractor 6 and the fourth tractor 7 move away from the transmission mechanism, and the first tractor 4 and the second tractor 5 move together close to the transmission mechanism. The sixth plane is formed by the output guide mechanism and the axis of the second driving shaft 2. The first dihedral angle is formed by the second plane and the fourth plane, and the second dihedral angle is formed by the fifth plane and the sixth plane. The first angle is a minimum value of a plane angle of the first dihedral angle and a plane angle of the second dihedral angle. The third dihedral angle is formed by the fourth plane and the sixth plane, and the fourth dihedral angle is formed by the first plane and the fifth plane. The second angle is a minimum value of a plane angle of the third dihedral angle and a plane angle of the fourth dihedral angle. Thus, the first tractor 4, the second tractor 5, the third tractor 6 and the fourth tractor 7 drive the instrument terminal to achieve a third degree of freedom movement. The proximal ends and the distal ends of the tractors will be specifically described below.

In this embodiment, the tractors are specifically described as follows.

The first tractor 4 comprises a first end, a first body 41 extending from the first end, and a second end connected to the first body 41; the first end (i.e., the proximal end of the first tractor 4) is wound around the first driving shaft 1 and partially fixedly connected to the first driving shaft 1; the first body 41 passes through one side of the first guide mechanism 8 and extends to the second end after the direction thereof is changed by the first output guide mechanism 12; and the second end (i.e., the distal end of the first tractor 4) is fixedly connected to the instrument terminal.

The second tractor 5 comprises a third end, a second body 51 extending from the third end, and a fourth end connected to the second body 51; the third end (i.e., the proximal end of the second tractor 5) is wound around the first driving shaft 1 in a direction opposite to the first end and is partially fixedly connected to the first driving shaft 1; the second body 51 passes through one side of the first guide mechanism 8 and extends to the fourth end after the direction thereof is changed by the first output guide mechanism 12; and the fourth end (i.e., the distal end of the second tractor 5) is fixedly connected to the instrument terminal, wherein the second body 51 and the first body 41 pass through the first guide mechanism 8 from a same side of the first guide mechanism 8. Preferably, the second body 51 and the first body 41 pass through the first guide mechanism 8 from the side of the first guide mechanism 8 away from the second driving shaft 2.

The third tractor 6 comprises a fifth end, a third body 61 extending from the fifth end, and a sixth end connected to the third body 61; the fifth end (i.e., the proximal end of the third tractor 6) is wound around the third driving shaft 3 and is partially fixedly connected to the third driving shaft 3; the third body 61 passes through one side of the second guide mechanism 9 and extends to the sixth end after the direction thereof is changed by the second output guide mechanism 13; and the sixth end (i.e., the distal end of the third tractor 6) is fixedly connected to the instrument terminal.

The fourth tractor 7 comprises a seventh end, a fourth body 71 extending from the seventh end, and an eighth end connected to the fourth body 71; the seventh end (i.e., the proximal end of the fourth tractor 7) is wound around the third driving shaft 3 in a direction opposite to the fifth end and is partially fixedly connected to the third driving shaft 3; the fourth body 71 passes through one side of the second guide mechanism 9 and extends to the eighth end after the direction thereof is changed by the second output guide mechanism 13; and the eighth end (i.e., the distal end of the fourth tractor 7) is fixedly connected to the instrument terminal. The fourth body 71 and the third body 61 pass through the second guide mechanism 9 from the same side of the second guide mechanism 9. Preferably, the fourth body 71 and the third body 61 pass through the second guide mechanism 9 from the side of the second guide mechanism 9 away from the second driving shaft 2.

Alternatively, in the axial direction, the second guide mechanism 9 is located above the first guide mechanism 8, and accordingly, the second output guide mechanism 13 is located above the first output guide mechanism 12. Each of the third body 61 and the fourth body 71, after being fixedly connected to the third driving shaft 3, passes over the first body 41 and the second body 51 (shown in FIG. 1) , is wound around the second guide mechanism 9 to change direction, and is fixedly connected to the instrument terminal after again passing over the first body 41 and the second body 51 and being wound around the second guide mechanism 9. Certainly, in the axial direction, the second guide mechanism 9 can also be located below the first guide mechanism 8, and accordingly, the second output guide mechanism 13 is located below the first output guide mechanism 12. Meanwhile, the third body 61 and the fourth body 71 after passing under the first body 41 and the second body 51 (not shown), is wound around the second guide mechanism 9 to change direction, and then are fixedly connected to the instrument terminal after again passing under the first body 41 and the second body 51 again and being wound to the second output guide mechanism 13. Here, it can be understood that the third body 61 and the fourth body 71 are always located on the same side of the first body 41 and the second body 51. In this way, the four tractors extend substantially in parallel and do not interfere with each other to ensure the reliability of the operation of the mechanism. In addition, the lengths of the four tractors wound around the corresponding driving shafts are determined according to the stroke of the instrument terminal, and the present invention does not make particular limitations herein.

In an example, the first guide mechanism 8 and the second guide mechanism 9 each comprise two coaxially mounted wheels, and the first output guide mechanism 12 and the second output guide mechanism 13 also each comprise two coaxially mounted wheels, and more preferably, each wheel is rotatable.

Preferably, the first driving shaft 1 and/or the third driving shaft 3 comprise rotators, i.e. a first rotator 14 and a second rotator 15. The first rotator 14 is locked on the first driving shaft 1, and the second rotator 15 is locked on third driving shaft 3. Moreover, the surface of each rotator is provided with two holes staggered in the circumferential direction. One end of the tractor is fixed in the hole after the body of the tractor is wound around and fixedly connected to the rotator of the corresponding driving shaft. For example, the first end is fixed in one hole of the first rotator 14 after being wound around the first rotator 14, and the third end is fixed in the one hole or another hole of the first rotator 14 after being wound around the first rotator 14 in a direction opposite to the first end. Similarly, the second rotator 15, the third driving shaft 3, the third tractor 6, and the fourth tractor 7 can be disposed based on the same principle.

### <Embodiment 2>

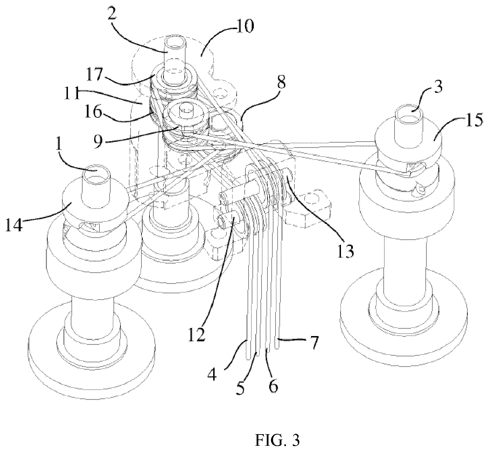

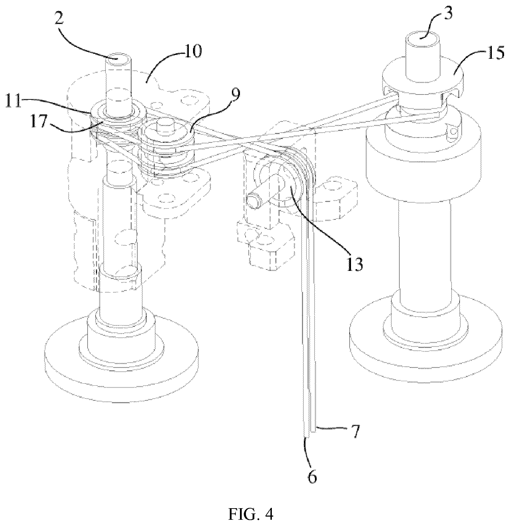

This embodiment differs from Embodiment 1 in that this embodiment has four guide mechanisms, and further includes two guide mechanisms apart from the first guide mechanism 8 and the second guide mechanism 9, i.e., a third guide mechanism 16 and a four guide mechanisms 17. Moreover, the winding mode of each tractor is correspondingly adjusted, specifically as shown in FIGs. 2-4, and the remaining settings of the transmission mechanism can be the same as those of Embodiment 1. FIG. 2 is an axial projection view of a transmission mechanism of Embodiment 1 of the present invention. FIG. 3 is a perspective view of the transmission mechanism shown in FIG. 2. FIG. 4 is a partial schematic view of the transmission mechanism shown in FIG. 3.

Specifically, the third guide mechanism 16 and the fourth guide mechanism 17 are sleeved on the second driving shaft 2, and the third guide mechanism 16 and the fourth guide mechanism 17 do not coincide in the axial direction of the second driving shaft 2, that is, located at different axial heights. The third guide mechanism 16 and the fourth guide mechanism 17 may be rotatable (i.e., being rotatably connected to the second driving shaft 2), and may also be fixedly connected to the second driving shaft 2 (i.e., being unable to rotate).

The first body 41, after being leaving the first driving shaft 1 and changing direction by passing through one side of both the first guide mechanism 8 and the third guide mechanism 16, extends to its second end which fixedly connected to the instrument terminal by further passing through the first output guide mechanism 12 . Accordingly, the second body 51, after being leaving the first driving shaft 1 in opposite direction and changing direction by passing through one side of both the first guide mechanism 8 and the third guide mechanism 16, extends to its fourth end which fixedly connected to the instrument terminal by further passing through the first output guide mechanism 12. Particularly, the first body 41 and the second body 51 pass through the first guide mechanism 8 from the side of the first guide mechanism 8 away from the second driving shaft 2. Similarly, the third body 61, after leaving the third driving shaft 3 and changing direction by passing through one side of both the second guide mechanism 9 and the fourth guide mechanism 17, extends to its sixth end which fixedly connected to the instrument terminal by further passing through the second output guide mechanism 13. Accordingly, the fourth body 71, after leaving the third driving shaft 3 in opposite direction and changing direction by passing through one side of both the second guide mechanism 9 and the fourth guide mechanism 17, extends to its eighth end which fixedly connected to the instrument terminal by further passing through the second output guide mechanism 13. Similarly, the third body 61 and the fourth body 71 pass through the second guide mechanism 9 from the side of the second guide mechanism 9 away from the second driving shaft 2.

As shown in FIGs. 2-4, if the second guide mechanism 9 is located above the first guide mechanism 8 along the axial direction and the fourth guide mechanism 17 is correspondingly located above the third guide mechanism 16; otherwise, if the second guide mechanism 9 is located below the first guide mechanism 8 along the axial direction, then the fourth guide mechanism 17 is located below the third guide mechanism 16.

Alternatively, the first body 41 and the second body 51 each pass through the same side of the first guide mechanism 8 and the third guide mechanism 16, and similarly, the third body 61 and the fourth body 71 each pass through the same side of the second guide mechanism 9 and the fourth guide mechanism 17. Here, the "same side" refers to a line connecting the tangent point formed by a corresponding tractor leaving a guide mechanism and the tangent point formed by a corresponding tractor entering the other guide mechanism and being parallel to the connecting line between the center points of such two guide mechanisms. For example, the connecting line between the tangent point formed by the first body 41 leaving the first guide mechanism 8 and the tangent point formed by the first body 41 entering the third guide mechanism 16 is parallel to the connecting line between the center points of the first guide mechanism 8 and the third guide mechanism 16 (the rotation center of the guide mechanism). Similar definitions are applicable to the other three bodies.

Then, other structural forms of the transmission mechanism according to Embodiment 2, are similar to those of the transmission mechanism according to Embodiment 1. Please refer to Embodiment 1 for details. Moreover, the working mode of the transmission mechanism according to Embodiment 2 is the same as that of the transmission mechanism according to Embodiment 1. That is, the first driving shaft 1 rotates to drive the first tractor 4 and the second tractor 5 to move in opposite directions, so as to drive the instrument terminal to achieve the first degree of freedom movement; the third driving shaft 3 rotates to drive the third tractor 6 and the fourth tractor 7 to move in opposite directions, so as to drive the instrument terminal to achieve the second degree of freedom movement; the second driving shaft 2 rotates to drive the four tractors to move simultaneously, and drive the instrument terminal to achieve the third degree of freedom movement.

### <Embodiment 3>

This embodiment differs from Embodiment 2 in that the third guide mechanism 16 and the fourth guide mechanism 17 of this embodiment are each disposed on a connector, i.e. disposed on the third connector 18 and the fourth connector 19, rather than sleeved on the second driving shaft 2. The third connector 18 and the fourth connector 19 are not intersected, and are each perpendicular to the axis of the second driving shaft 2. Moreover, the fourth guide mechanism 17 is farther from the first driving shaft 1 than the third guide mechanism 16, and the third guide mechanism 16 is farther from the third driving shaft 3 than the fourth guide mechanism 17.

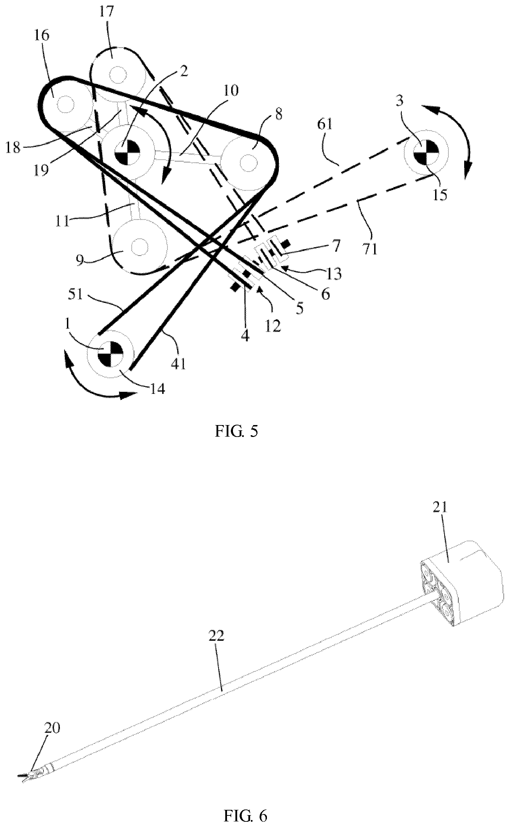

One end of the third connector 18 is fixed on the second driving shaft 2, and the other end of the third connector 18 is connected to the third guide mechanism 16. One end of the fourth connector 19 is fixed on the second driving shaft 2, and the other end is connected to the fourth guide mechanism 17. Please refer to FIG. 5, an axial projection view of the transmission mechanism according to Embodiment 3 of the present invention, for details.

In FIG. 5, the first connector 10, the second connector 11, the third connector 18 and the fourth connector 19 are sequentially spaced and distributed in the circumferential direction and distributed in the axial direction in a staggered manner to prevent interference between the tractors. For example the first connector 10 and the third connector 18 are at the same height of the second driving shaft 2, with the second connector 11 and the fourth connector 19 being at the same height of the second driving shaft 2, and the first connector 10 and the second connectors 11 are located at different heights of the second driving shaft 2 (that is, the third connector 18 does not coincide with the fourth connector 19 in the axial direction of the second driving shaft 2); or the first connector 10 and the third connector 18 are disposed adjacent to each other in the axial direction, with the second connector 11 and the fourth connector 19 being disposed adjacent to each other in the axial direction, but the first connector 10 and the third connector 18 are spaced apart from the second connector 11 and the fourth connector 19 by a large axial distance to facilitate the extension of the tractors. Further,, the first body 41, after leaving the first driving shaft 1 and changing direction by passing through the side of each of the first guide mechanism 8 and the third guide mechanism 16 away from the second driving shaft 2, extends to its second end which fixedly connected to the instrument terminal by further passing through the first output guide mechanism 12. Accordingly, the second body 51, after changing direction by passing through the side of each of the first guide mechanism 8 and the third guide mechanism 16 away from the second driving shaft 2, extends to its fourth end which fixedly connected to the instrument terminal by further passing through the first output guide mechanism 12. Similarly, the third body 61, after leaving the third driving shaft 3 and changing direction by passing through the side of each of the second guide mechanism 9 and the fourth guide mechanism 17 away from the second driving shaft 2, extends to its sixth end which fixedly connected to the instrument terminal by further passing through the second output guide mechanism 13 Similarly, the fourth body 71, after changing direction by passing through one side of each of the second guide mechanism 9 and the fourth guide mechanism 17 away from the second driving shaft 2, extends to its eighth end which fixedly connected to the instrument terminal by further passing through the second output guide mechanism 13.

In FIG. 5, both of the first body 41 and the second body 51 pass through the same side of the first guide mechanism 8 and the third guide mechanism 16, and similarly, both of the third body 61 and the fourth body 71 pass through the same side of the second guide mechanism 9 and the fourth guide mechanism 17. Here, the "same side" in this embodiment has a same definition as in Embodiment 2. The "same side" refers to a connecting line connecting the tangent point formed by a corresponding tractor leaving a guide mechanism and the tangent point formed by a corresponding tractor entering the other guide mechanism and being parallel to the connecting line between the center points of such two guide mechanisms. For example, the connecting line between the tangent point formed by the first body 41 leaving the first guide mechanism 8 and the tangent point formed by the first body 41 entering the third guide mechanism 16 is parallel to the connecting line between the center points of the first guide mechanism 8 and the third guide mechanism 16 (the rotation center of the guide mechanism). Similar definitions are applicable to the other three bodies.

In conclusion, different configurations of the transmission mechanism are described in detail through the foregoing embodiments.

Those skilled in the art can draw inferences about other cases from one instance according to the contents of the foregoing embodiments.

The transmission mechanism provided by the present invention, when used to drive the instrument terminal of the surgical instrument to move, involves four tractors, three driving shafts and guide mechanisms, thereby enabling a decreased number of selected parts, and further resulting in a low cost.

Moreover, the transmission mechanism of the present invention is able to achieve three degrees of freedom movement of the instrument terminal by rotating three driving shafts to drive the corresponding four tractors to move, thereby having a simple structure. Compared with the existing transmission mechanism using six wire ropes (here, in the prior art, the realization of one degree of freedom movement at the instrument terminal generally requires two wire ropes for driving, then the realization of three degrees of freedom movement at the end of the instrument requires six wire ropes), using four tractors to achieve three degrees of freedom movement is able to reduce the number of wire ropes, minimizes the dimension, simplify the driving control, and thus allow the surgical instruments to be made smaller, which in turn enables a smaller minimally invasive wound to alleviate the patient's pain and also an improved flexibility of the surgical operation due to the more flexible movement of the surgical instrument in the small space of the human body.

It should be understood by those skilled in the art that the transmission mechanism of the present invention realizes the movement access of three joints of the four tractors through movement decoupling of the four tractors, so that the movement of the three joints that drive the instrument terminal is achieved by the movement of the decoupled four tractors.

Further, each of the foregoing tractors is a wire, a rope, a belt, etc., which is not limited in the present invention.

Furthermore, based on the foregoing transmission mechanism, this embodiment further provides a surgical instrument, comprising the transmission mechanism provided in the foregoing embodiments and configured to drive the instrument terminal 20 located at the end of the surgical instrument to move in three degrees of freedom. The so-called "terminal " refers to an end away from the product operator. Since the surgical instrument of this embodiment employs the transmission mechanism of the foregoing embodiments, the advantageous effects of the surgical instrument brought by the transmission mechanism can be referred to the foregoing embodiments.

Here, due to the compact layout of the transmission mechanism, the mounting space of the surgical instrument is able to be effectively saved, so that the dimension of the surgical instrument is able to be smaller, and the stability and reliability of the surgical instrument are able to be effectively ensured. In addition, the weight of the surgical instrument is also greatly reduced based on the tractor transmission, which meets the requirements of lightweight development.

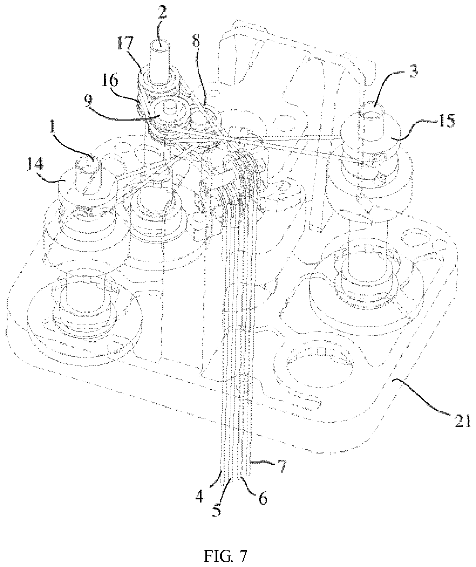

FIG. 6 is a perspective view of a surgical instrument according to an embodiment of the present invention. FIG. 7 is a perspective view of a transmission mechanism in an instrument set according to an embodiment of the present invention. As shown in FIGs. 6 and 7, the surgical instrument further includes an instrument set 21, configured to fix the transmission mechanism by disposing the foregoing transmission mechanism into the instrument set 21.

The surgical instrument further includes a tube 22. The tube 22 is connected to the instrument set 21 and the instrument terminal 20 respectively. All the tractors, after changing directions by passing through the guide mechanisms of the transmission mechanism, are fixedly connected to the instrument terminal 20 through the tube 22.

In addition, the instrument terminal according to one embodiment includes a first joint, a second joint and a third joint. The first joint are driven by the first tractor 4 and the second tractor 5 to realize the first degree of freedom movement. The second joint is driven by the third tractor 6 and the fourth tractor 7 to realize the second degree of freedom movement. The third joint is driven by the first tractor 4, the second tractor 5, the third tractor 6 and the fourth tractor 7 to realize the third degree of freedom movement.

The above description is only a description of the preferred embodiments of the present invention, rather than any limitations to the scope of the present invention.

## Claims

1. A transmission mechanism disposed on a surgical instrument and configured to drive an instrument terminal (20) located at an end of the surgical instrument to move, wherein the transmission mechanism comprises:
driving shafts (1, 2, 3) comprising a first driving shaft (1), a second driving shaft (2) and a third driving shaft (3) which are arranged in parallel;
tractors (4, 5, 6, 7) comprising a first tractor (4), a second tractor (5), a third tractor (6) and a fourth tractor (7); and
a guide mechanism (8, 9, 16, 17);
wherein the first tractor (4) and the second tractor (5) are wound around the first driving shaft (1) in opposite directions, partially fixedly connected to the first driving shaft (1), and fixedly connected to the instrument terminal (20) after changing directions via the guide mechanism (8, 9, 16, 17); and the third tractor (6) and the fourth tractor (7) are wound around the third driving shaft (3) in opposite directions, partially fixedly connected to the third driving shaft (3), and fixedly connected to the instrument terminal (20) after changing directions via the guide mechanism (8, 9);
**characterised in that**
the guide mechanism (8, 9, 16, 17) is configured such that when the second driving shaft (2) rotates, a movement direction of portions of the first tractor (4) and the second tractor (5) located between the guide mechanism (8, 9) and the instrument terminal (20) is opposite to a movement direction of portions of the third tractor (6) and the fourth tractor (7) located between the guide mechanism (8, 9) and the instrument terminal (20).

2. The transmission mechanism according to claim 1, wherein the guide mechanism (8, 9, 16, 17) comprises a first guide mechanism (8) and a second guide mechanism (9), with axes of the first guide mechanism (8) and the second guide mechanism (9) being parallel to axes of the driving shafts (1, 2, 3);
wherein the guide mechanism (8, 9, 16, 17) is located in a first space defined by a first plane formed by axes of the first driving shaft (1) and the second driving shaft (2), and a second plane formed by axes of the second driving shaft (2) and the third driving shaft (3); the first guide mechanism (8) is farther from the first driving shaft (1) than the second guide mechanism (9), and the second guide mechanism (9) is farther from the third driving shaft (3) than the first guide mechanism (8);
wherein the first tractor (4) and the second tractor (5), wound around the first driving shaft (1) in opposite directions and partially fixedly connected to the first driving shaft (1), are fixedly connected to the instrument terminal (20) after changing directions by passing through a same side of the first guide mechanism (8); and the third tractor (6) and the fourth tractor (7), wound around the third driving shaft (3) in opposite directions and partially fixedly connected to the third driving shaft (3), are fixedly connected to the instrument terminal (20) after changing directions by passing through a same side of the second guide mechanism (9).

3. The transmission mechanism according to claim 2, further comprising:
Connectors (10, 11, 18, 19), comprising a first connector (10) and a second connector (11);
wherein the first connector (10) and the second connector (11) are not intersected, and are each perpendicular to the axis of the second driving shaft (2); one end of the first connector (10) is fixedly connected to the second driving shaft (2) with the other end of the first connector (10) being fixedly connected to the first guide mechanism (8); and one end of the second connector (11) is fixedly connected to the second driving shaft (2), with the other end of the second connector (11) being fixedly connected to the second guide mechanism (9).

4. The transmission mechanism according to claim 2, further comprising:
an output guide mechanism (12, 13), comprising a first output guide mechanism (12) and a second output guide mechanism (13);
wherein the output guide mechanism (12, 13) is located in a second space defined by a third plane formed by axes of the first driving shaft (1) and the third driving shaft (3), a fourth plane formed by axes of the second driving shaft (2) and the first guide mechanism (8), and a fifth plane formed by axes of the second driving shaft (2) and the second guide mechanism (9); the axes of the first output guide mechanism (12) and the second output guide mechanism (13) are perpendicular to the axis of the second driving shaft (2), and the axes of the first output guide mechanism (12) and the second output guide mechanism (13) are not intersected;
wherein the first tractor (4) and the second tractor (5), after changing directions by passing through a same side of the first guide mechanism (8), are fixedly connected to the instrument terminal (20) by further passing through the first output guide mechanism (12); and the third tractor (6) and the fourth tractor (7), after changing directions by passing through the same side of the second guide mechanism (9), are fixedly connected to the instrument terminal (20) by further passing through the second output guide mechanism (13).

5. The transmission mechanism according to claim 4, wherein the guide mechanism (8, 9, 16, 17) further comprises a third guide mechanism (16) and a fourth guide mechanism (17) which are sleeved on the second driving shaft (2); and the third guide mechanism (16) does not coincide with the fourth guide mechanism (17) in an axial direction of the second driving shaft (2);
the first tractor (4) and the second tractor (5), after being wound around the first driving shaft (1) and changing directions by passing through one side of both the first guide mechanism (8) and the third guide mechanism (16), are fixedly connected to the instrument terminal (20) by further passing through the first output guide mechanism (12); and the third tractor (6) and the fourth tractor (7), after being wound around the third driving shaft (3) and changing directions by passing through one side of both the second guide mechanism (9) and the fourth guide mechanism (17), are fixedly connected to the instrument terminal (20) by further passing through the second output guide mechanism (13).

6. The transmission mechanism according to claim 4, wherein the guide mechanism further comprises a third mechanism (16) and a fourth guide mechanism (17); the fourth guide mechanism (17) is farther from the first driving shaft (1) than the third guide mechanism (16), and the third guide mechanism (16) is farther from the third driving shaft (3) than the fourth guide mechanism (17);
wherein the first tractor (4) and the second tractor (5), after being wound around the first driving shaft (1) and changing directions by passing through one side of both the first guide mechanism (8) and the third guide mechanism (16), are fixedly connected to the instrument terminal (20) by further passing through the first output guide mechanism (12); and the third tractor (6) and the fourth tractor (7), after being wound around the third driving shaft (3) and changing directions by passing through one side of both the second guide mechanism (9) and the fourth guide mechanism (17), are fixedly connected to the instrument terminal (20) by further passing through the second output guide mechanism (13).

7. The transmission mechanism according to claim 6, further comprising:
connectors (10, 11, 18, 19) comprising a third connector (18) and a fourth connector (19), wherein the third connector (18) and the fourth connector (19) are not intersected, and are each perpendicular to the axis of the second driving shaft (2); one end of the third connector (18) is fixedly connected to the second driving shaft (2) with the other end of the third connector (18) being fixedly connected to the third guide mechanism (16); and one end of the fourth connector (19) is fixedly connected to the second driving shaft (2), with the other end of the fourth connector (19) being fixedly connected to the fourth guide mechanism (17).

8. The transmission mechanism according to claim 5 or 6, wherein the first tractor (4) and the second tractor (5) pass through the same side of the first guide mechanism (8) and the third guide mechanism (16), and the third tractor (6) and the fourth tractor (7) pass through the same side of the second guide mechanism (9) and the fourth guide mechanism (17).

9. The transmission mechanism according to any one of claims 1-7, wherein a distance between the axis of the second driving shaft (2) and the axis of the first driving shaft (1) is equal to a distance between the axis of the second driving shaft (2) and the axis of the third driving shaft (3).

10. The transmission mechanism according to any one of claims 2-7, wherein the first tractor (4) and the second tractor (5) pass through the first guide mechanism (8) from a side of the first guide mechanism (8) away from the second driving shaft (2), and the third tractor (6) and the fourth tractor (7) pass through the second guide mechanism (9) from a side of the second guide mechanism (9) away from the second driving shaft (2).

11. The transmission mechanism according to any one of claim 4-7, wherein the output guide mechanism is located on a mid-vertical plane of the third plane, or the first output guide mechanism (12) and the second output guide mechanism (13) are symmetrically disposed with respect to the mid-vertical plane.

12. The transmission mechanism according to any one of claim 4-7, wherein the first tractor (4) comprises a first end, a first body (41) extending from the first end, and a second end connected to the first body (41); the first end is wound around the first driving shaft (1) and is partially fixedly connected to the first driving shaft (1); the first body (41) passes through one side of the guide mechanism (8, 9), and extends to the second end after a direction thereof is changed by the first output guide mechanism (12); and the second end is fixedly connected to the instrument terminal (20);
the second tractor (5) comprises a third end, a second body (51) extending from the third end, and a fourth end connected to the second body (51); the third end is wound around the first driving shaft (1) in a direction opposite to the first end, and is partially fixedly connected to the first driving shaft (1); the second body (51) passes through one side of the guide mechanism (8, 9), and extends to the fourth end after a direction thereof is changed by the first output guide mechanism (12); and the fourth end is fixedly connected to the instrument terminal (20).

13. The transmission mechanism according to any one of claims 4-7, wherein the third tractor (6) comprises a fifth end, a third body (61) extending from the fifth end, and a sixth end connected to the third body (61); the fifth end is wound around the third driving shaft (3) and is partially fixedly connected to the third driving shaft (3); the third body (61) passes through one side of the guide mechanism (8, 9), and extends to the sixth end after a direction thereof is changed by the second output guide mechanism (13); and the sixth end is fixedly connected to the instrument terminal (20);
the fourth tractor (7) comprises a seventh end, a fourth body (71) extending from the seventh end, and an eighth end connected to the fourth body (71); the seventh end is wound around the third driving shaft (3) in a direction opposite to the fifth end, and is partially fixedly connected to the third driving shaft (3); the fourth body (71) passes through one side of the guide mechanism (8, 9), and extends to the eighth end after a direction thereof is changed by the second output guide mechanism (13); the eighth end is fixedly connected to the instrument terminal (20); and the third body (61) and the fourth body (71) are always located on a same side of the first body (41) and the second body (51).

14. The transmission mechanism according to any one of claims 1-7, wherein the first driving shaft (1) and/or the third driving shaft (3) further comprises a rotator (14, 15); the rotator (14, 15) is coaxially fixedly connected to the corresponding driving shaft (1, 2, 3) ; the rotator (14, 15) is provided with a hole; and the tractor (4, 5, 6, 7) is wound around the rotator (14, 15) of the corresponding driving shaft (1, 2, 3) and is fixedly connected to the hole of the rotator (14, 15).

15. A surgical instrument, comprising an instrument terminal (20) located at an end of the surgical instrument and the transmission mechanism according to any one of claims 1-14, wherein the transmission mechanism is configured to drive the instrument terminal (20) to move.

## Patentansprüche

1. Übertragungsmechanismus, der an einem Operationsinstrument angeordnet ist und ausgebildet ist, einen Instrumentenanschluss (20) zur Bewegung anzutreiben, der sich an einem Ende des Operationsinstruments befindet, wobei der Übertragungsmechanismus folgendes umfasst:
Antriebsschäfte (1, 2, 3), umfassend einen ersten Antriebsschaft (1), einen zweiten Antriebsschaft (2) und einen dritten Antriebsschaft (3), welche parallel angeordnet sind;
Zugvorrichtungen (4, 5, 6, 7), umfassend eine erste Zugvorrichtung (4), eine zweite Zugvorrichtung (5), eine dritte Zugvorrichtung (6) und eine vierte Zugvorrichtung (7); und
einen Führungsmechanismus (8, 9, 16, 17);
wobei die erste Zugvorrichtung (4) und die zweite Zugvorrichtung (5) um den ersten Antriebsschaft (1) in entgegengesetzten Richtungen gewickelt, teilweise fest mit dem ersten Antriebsschaft (1) verbunden und nach einem Richtungswechsel über den Führungsmechanismus (8, 9, 16, 17) fest mit dem Instrumentenanschluss (20) verbunden sind; und wobei die dritte Zugvorrichtung (6) und die vierte Zugvorrichtung (7) um den dritten Antriebsschaft (3) in entgegengesetzten Richtungen gewickelt, teilweise fest mit dem dritten Antriebsschaft (3) verbunden und nach einem Richtungswechsel über den Führungsmechanismus (8, 9) fest mit dem Instrumentenanschluss (20) verbunden sind;
**dadurch gekennzeichnet, dass**
der Führungsmechanismus (8, 9, 16, 17) so ausgebildet ist, dass, wenn sich der zweite Antriebsschaft (2) dreht, eine Bewegungsrichtung von Abschnitten der ersten Zugvorrichtung (4) und der zweiten Zugvorrichtung (5), die sich zwischen dem Führungsmechanismus (8, 9) und dem Instrumentenanschluss (20) befinden, entgegengesetzt zu einer Bewegungsrichtung von Abschnitten der dritten Zugvorrichtung (6) und der vierten Zugvorrichtung (7) ist, die sich zwischen dem Führungsmechanismus (8, 9) und dem Instrumentenanschluss befinden (20).

2. Übertragungsmechanismus nach Anspruch 1, wobei der Führungsmechanismus (8, 9, 16, 17) einen ersten Führungsmechanismus (8) und einen zweiten Führungsmechanismus (9) umfasst, wobei Achsen des ersten Führungsmechanismus (8) und des zweiten Führungsmechanismus (9) parallel zu den Achsen der Antriebsschäfte (1, 2, 3) sind;
wobei der Führungsmechanismus (8, 9, 16, 17) in einem ersten Raum gelegen ist, der durch eine erste Ebene definiert ist, die durch Achsen des ersten Antriebsschaft (1) und des zweiten Antriebsschaft (2) gebildet ist, und eine zweite Ebene durch Achsen des zweiten Antriebsschafts (2) und des dritten Antriebsschafts (3) gebildet ist; wobei der erste Führungsmechanismus (8) entfernter von dem ersten Antriebsschaft (1) als der zweite Führungsmechanismus (9) ist, und wobei der zweite Führungsmechanismus (9) entfernter von dem dritten Antriebsschaft (3) als der erste Führungsmechanismus (8) ist;
wobei die erste Zugvorrichtung (4) und die zweite Zugvorrichtung (5), welche um den ersten Antriebsschaft (1) in entgegengesetzten Richtungen gewickelt und mit dem ersten Antriebsschaft (1) teilweise fest verbunden sind, nach einem Richtungswechsel durch Durchlaufen einer gleichen Seite des ersten Führungsmechanismus (8) fest mit dem Instrumentenanschluss (20) verbunden sind; und wobei die dritte Zugvorrichtung (6) und die vierte Zugvorrichtung (7), welche um den dritten Antriebsschaft (3) in entgegengesetzten Richtungen gewickelt und mit dem dritten Antriebsschaft (3) teilweise fest verbunden sind, nach einem Richtungswechsel durch Durchlaufen einer gleichen Seite des zweiten Führungsmechanismus (9) fest mit dem Instrumentenanschluss (20) verbunden sind.

3. Übertragungsmechanismus nach Anspruch 2, ferner umfassend:
Verbinder (10, 11, 18, 19), umfassend einen ersten Verbinder (10) und einen zweiten Verbinder (11);
wobei der erste Verbinder (10) und der zweite Verbinder (11) nicht überschnitten werden und jeweils senkrecht zur Achse des zweiten Antriebsschafts (2) sind; wobei ein Ende des ersten Verbinders (10) mit dem zweiten Antriebsschaft (2) fest verbunden ist, wobei das andere Ende des ersten Verbinders (10) mit dem ersten Führungsmechanismus (8) fest verbunden ist; und wobei ein Ende des zweiten Verbinders (11) mit dem zweiten Antriebsschaft (2) fest verbunden ist, wobei das andere Ende des zweiten Verbinders (11) mit dem zweiten Führungsmechanismus (9) fest verbunden ist.

4. Übertragungsmechanismus nach Anspruch 2, ferner umfassend:
einen Ausgabeführungsmechanismus (12, 13), umfassend einen ersten Ausgabeführungsmechanismus (12) und einen zweiten Ausgabeführungsmechanismus (13);
wobei der Ausgabeführungsmechanismus (12, 13) in einem zweiten Raum gelegen ist, der definiert ist durch eine dritte Ebene, die durch Achsen des ersten Antriebsschafts (1) und des dritten Antriebsschafts (3) gebildet ist, eine vierte Ebene, die durch Achsen des zweiten Antriebsschafts (2) und dem ersten Führungsmechanismus (8) gebildet ist, und eine fünfte Ebene, die durch Achsen des zweiten Antriebsschafts (2) und des zweiten Führungsmechanismus (9) gebildet ist; wobei die Achsen des ersten Ausgabeführungsmechanismus (12) und des zweiten Ausgabeführungsmechanismus (13) senkrecht zur Achse des zweiten Antriebsschafts (2) sind und die Achsen des ersten Ausgabeführungsmechanismus (12) und des zweiten Ausgabeführungsmechanismus (13) nicht überschnitten werden;
wobei die erste Zugvorrichtung (4) und die zweite Zugvorrichtung (5), nach einem Richtungswechsel durch Durchlaufen einer gleichen Seite des ersten Führungsmechanismus (8) mit dem Instrumentenanschluss (20) durch weiteres Durchlaufen durch den ersten Ausgabeführungsmechanismus (12) fest verbunden sind; und wobei die dritte Zugvorrichtung (6) und die vierte Zugvorrichtung (7), nach einem Richtungswechsel durch Durchlaufen einer gleichen Seite des zweiten Führungsmechanismus (9) mit dem Instrumentenanschluss (20) durch weiteres Durchlaufen durch den zweiten Ausgabeführungsmechanismus (13) fest verbunden sind.

5. Übertragungsmechanismus nach Anspruch 4, wobei der Führungsmechanismus (8, 9, 16, 17) ferner einen dritten Führungsmechanismus (16) und einen vierten Führungsmechanismus (17) umfasst, die auf dem zweiten Antriebsschaft (2) aufgehülst sind; und wobei der dritte Führungsmechanismus (16) nicht mit dem vierten Führungsmechanismus (17) in einer axialen Richtung des zweiten Antriebsschafts (2) zusammenfällt;
wobei die erste Zugvorrichtung (4) und die zweite Zugvorrichtung (5), nachdem diese um den ersten Antriebsschaft (1) gewickelt sind und durch Durchlaufen einer Seite sowohl des ersten Führungsmechanismus (8) als auch des dritten Führungsmechanismus (16) Richtungen gewechselt haben, mit dem Instrumentenanschluss (20) durch weiteres Durchlaufen durch den ersten Ausgabeführungsmechanismus (12) fest verbunden sind; und wobei die dritte Zugvorrichtung (6) und die vierte Zugvorrichtung (7), nachdem diese um den dritten Antriebsschaft (3) gewickelt sind und durch Durchlaufen einer Seite sowohl des zweiten Führungsmechanismus (9) als auch des vierten Führungsmechanismus (17) Richtungen gewechselt haben, mit dem Instrumentenanschluss (20) durch weiteres Durchlaufen durch den zweiten Ausgabeführungsmechanismus (13) fest verbunden sind.

6. Übertragungsmechanismus nach Anspruch 4, wobei der Führungsmechanismus ferner einen dritten Mechanismus (16) und einen vierten Führungsmechanismus (17) umfasst; wobei der vierte Führungsmechanismus (17) entfernter von dem ersten Antriebsschaft (1) als der dritte Führungsmechanismus (16) ist, und wobei der dritte Führungsmechanismus (16) entfernter von dem dritten Antriebsschaft (3) als der vierte Führungsmechanismus (17) ist;
wobei die erste Zugvorrichtung (4) und die zweite Zugvorrichtung (5), nachdem diese um den ersten Antriebsschaft (1) gewickelt sind und durch Durchlaufen einer Seite sowohl des ersten Führungsmechanismus (8) als auch des dritten Führungsmechanismus (16) Richtungen gewechselt haben, mit dem Instrumentenanschluss (20) durch weiteres Durchlaufen durch den ersten Ausgabeführungsmechanismus (12) fest verbunden sind; und wobei die dritte Zugvorrichtung (6) und die vierte Zugvorrichtung (7), nachdem diese um den dritten Antriebsschaft (3) gewickelt sind und durch Durchlaufen einer Seite sowohl des zweiten Führungsmechanismus (9) als auch des vierten Führungsmechanismus (17) Richtungen gewechselt haben, mit dem Instrumentenanschluss (20) durch weiteres Durchlaufen durch den zweiten Ausgabeführungsmechanismus (13) fest verbunden sind.

7. Übertragungmechanismus nach Anspruch 6, ferner umfassend:
Verbinder (10, 11, 18, 19), umfassend einen dritten Verbinder (18) und einen vierten Verbinder (19), wobei der dritte Verbinder (18) und der vierte Verbinder (19) nicht überschnitten werden und jeweils senkrecht zur Achse des zweiten Antriebsschafts (2) sind; wobei ein Ende des dritten Verbinders (18) mit dem zweiten Antriebsschaft (2) fest verbunden ist, wobei das andere Ende des dritten Verbinders (18) mit dem dritten Führungsmechanismus (16) fest verbunden ist; und wobei ein Ende des vierten Verbinders (19) mit dem zweiten Antriebsschaft (2) fest verbunden ist, wobei das andere Ende des vierten Verbinders (19) mit dem vierten Führungsmechanismus (17) fest verbunden ist.

8. Übertragungsmechanismus nach Anspruch 5 oder 6, wobei die erste Zugvorrichtung (4) und die zweite Zugvorrichtung (5) dieselbe Seite des ersten Führungsmechanismus (8) und des dritten Führungsmechanismus (16) durchlaufen, und wobei die dritte Zugvorrichtung (6) und die vierte Zugvorrichtung (7) dieselbe Seite des zweiten Führungsmechanismus (9) und des vierten Führungsmechanismus (17) durchlaufen.

9. Übertragungsmechanismus nach einem der Ansprüche 1-7, wobei ein Abstand zwischen der Achse des zweiten Antriebsschafts (2) und der Achse des ersten Antriebsschafts (1) gleich einem Abstand zwischen der Achse des zweite Antriebsschafts (2) und die Achse des dritten Antriebsschafts ist (3).

10. Übertragungsmechanismus nach einem der Ansprüche 2-7, wobei die erste Zugvorrichtung (4) und die zweite Zugvorrichtung (5) den ersten Führungsmechanismus (8) von einer Seite des ersten Führungsmechanismus (8) abseits des zweiten Antriebsschafts (2) durchlaufen, und wobei die dritte Zugvorrichtung (6) und die vierte Zugvorrichtung (7) den zweiten Führungsmechanismus (9) von einer Seite des zweiten Führungsmechanismus (9) abseits des zweiten Antriebsschafts (2) durchlaufen.

11. Übertragungsmechanismus nach einem der Ansprüche 4-7, wobei der Ausgabeführungsmechanismus auf einer mittelvertikalen Ebene der dritten Ebene gelegen ist, oder wobei der erste Ausgabeführungsmechanismus (12) und der zweite Ausgabeführungsmechanismus (13) bezüglich der mittelvertikalen Ebene symmetrisch angeordnet sind.

12. Übertragungsmechanismus nach einem der Ansprüche 4-7, wobei die erste Zugvorrichtung (4) ein erstes Ende umfasst, wobei ein erster Körper (41) sich von dem ersten Ende erstreckt, und ein zweites Ende umfasst, das mit dem ersten Körper (41) verbunden ist; wobei das erste Ende um den ersten Antriebsschaft (1) gewickelt ist und mit dem ersten Antriebsschaft (1) teilweise fest verbunden ist; wobei der erste Körper (41) eine Seite des Führungsmechanismus (8, 9) durchläuft und nach einer Richtungsänderung desselben durch den ersten Ausgabeführungsmechanismus (12) sich zum zweiten Ende erstreckt; und wobei das zweite Ende mit dem Instrumentenanschluss (20) fest verbunden ist;
wobei die zweite Zugvorrichtung (5) ein drittes Ende umfasst, wobei ein zweiter Körper (51) sich von dem dritten Ende erstreckt, und ein viertes Ende umfasst, das mit dem zweiten Körper (51) verbunden ist; wobei das dritte Ende um den ersten Antriebsschaft (1) mit einer dem ersten Ende entgegengesetzten Richtung gewickelt ist und mit dem ersten Antriebsschaft (1) teilweise fest verbunden ist; wobei der zweite Körper (51) eine Seite des Führungsmechanismus (8, 9) durchläuft und nach einer Richtungsänderung desselben durch den ersten Ausgabeführungsmechanismus (12) sich zum vierten Ende erstreckt; und wobei das vierte Ende mit dem Instrumentenanschluss (20) fest verbunden ist.

13. Übertragungsmechanismus nach einem der Ansprüche 4-7, wobei die dritte Zugvorrichtung (6) ein fünftes Ende umfasst, wobei ein dritter Körper (61) sich von dem fünften Ende erstreckt, und ein sechstes Ende umfasst, das mit dem dritten Körper (61) verbunden ist; wobei das fünfte Ende um den dritten Antriebsschaft (3) gewickelt ist und mit dem dritten Antriebsschaft (3) teilweise fest verbunden ist; wobei der dritte Körper (61) eine Seite des Führungsmechanismus (8, 9) durchläuft und nach einer Richtungsänderung desselben durch den zweiten Ausgabeführungsmechanismus (13) sich zum sechsten Ende erstreckt; und wobei das sechste Ende mit dem Instrumentenanschluss (20) fest verbunden ist;
wobei die vierte Zugvorrichtung (7) ein siebtes Ende umfasst, wobei ein vierter Körper (71) sich von dem siebten Ende erstreckt, und ein achtes Ende umfasst, das mit dem vierten Körper (71) verbunden ist; wobei das siebte Ende um den dritten Antriebsschaft (3) mit einer Richtung entgegengesetzt zu dem fünften Ende gewickelt ist und mit dem dritten Antriebsschaft (3) teilweise fest verbunden ist; wobei der vierte Körper (71) eine Seite des Führungsmechanismus (8, 9) durchläuft und nach einer Richtungsänderung desselben durch den zweiten Ausgabeführungsmechanismus (13) sich zum achten Ende erstreckt; wobei das achte Ende mit dem Instrumentenanschluss (20) fest verbunden ist; und wobei der dritte Körper (61) und der vierte Körper (71) immer auf einer selben Seite des ersten Körpers (41) und des zweiten Körpers (51) gelegen sind.

14. Übertragungsmechanismus nach einem der Ansprüche 1-7, wobei der erste Antriebsschaft (1) und/oder der dritte Antriebsschaft (3) ferner einen Rotator (14, 15) umfassen; wobei der Rotator (14, 15) mit dem entsprechenden Antriebsschaft (1, 2, 3) koaxial fest verbunden ist; wobei der Rotator (14, 15) mit einem Loch versehen ist; und wobei die Zugvorrichtung (4, 5, 6, 7) um den Rotator (14, 15) des entsprechenden Antriebsschafts (1, 2, 3) gewickelt ist und fest mit dem Loch des Rotators (14, 15) verbunden ist.

15. Operationsinstrument, umfassend einen Instrumentenanschluss (20), der an einem Ende des Operationsinstruments gelegen ist, und den Übertragungsmechanismus nach einem der Ansprüche 1 bis 14, wobei der Übertragungsmechanismus ausgebildet ist, den Instrumentenanschluss (20) zu bewegen.

## Revendications

1. Mécanisme de transmission disposé sur un instrument chirurgical et conçu pour amener un organe terminal d'instrument (20) situé à une extrémité de l'instrument chirurgical à effectuer des mouvements, le mécanisme de transmission comprenant :
des arbres d'entraînement (1, 2, 3) comprenant un premier arbre d'entraînement (1), un deuxième arbre d'entraînement (2) et un troisième arbre d'entraînement (3) qui sont agencés parallèlement ;
des éléments de traction (4, 5, 6, 7) comprenant un premier élément de traction (4), un deuxième élément de traction (5), un troisième élément de traction (6) et un quatrième élément de traction (7) ; et
un mécanisme de guidage (8, 9, 16, 17) ;
le premier élément de traction (4) et le deuxième élément de traction (5) étant enroulés autour du premier arbre d'entraînement (1) dans des directions opposées, partiellement raccordés de manière fixe au premier arbre d'entraînement (1) et raccordés de manière fixe à l'organe terminal d'instrument (20) après des changements de direction par le biais du mécanisme de guidage (8, 9, 16, 17) ; et le troisième élément de traction (6) et le quatrième élément de traction (7) étant enroulés autour du troisième arbre d'entraînement (3) dans des directions opposées, partiellement raccordés de manière fixe au troisième arbre d'entraînement (3) et raccordés de manière fixe à l'organe terminal d'instrument (20) après des changements de direction par le biais du mécanisme de guidage (8, 9) ;
**caractérisé en ce que**
le mécanisme de guidage (8, 9, 16, 17) est conçu de telle sorte que, lorsque le deuxième arbre d'entraînement (2) tourne, une direction de mouvement de parties du premier élément de traction (4) et du deuxième élément de traction (5) situées entre le mécanisme de guidage (8, 9) et l'organe terminal d'instrument (20) est l'inverse d'une direction de mouvement de parties du troisième élément de traction (6) et du quatrième élément de traction (7) situées entre le mécanisme de guidage (8, 9) et l'organe terminal d'instrument (20).

2. Mécanisme de transmission selon la revendication 1, dans lequel le mécanisme de guidage (8, 9, 16, 17) comprend un premier mécanisme de guidage (8) et un deuxième mécanisme de guidage (9), des axes du premier mécanisme de guidage (8) et du deuxième mécanisme de guidage (9) étant parallèles aux axes des arbres d'entraînement (1, 2, 3) ;
dans lequel le mécanisme de guidage (8, 9, 16, 17) est situé dans un premier espace défini par un premier plan formé par des axes du premier arbre d'entraînement (1) et du deuxième arbre d'entraînement (2), et un deuxième plan formé par des axes du deuxième arbre d'entraînement (2) et du troisième arbre d'entraînement (3) ; le premier mécanisme de guidage (8) se trouve plus loin du premier arbre d'entraînement (1) que le deuxième mécanisme de guidage (9), et le deuxième mécanisme de guidage (9) se trouve plus loin du troisième arbre d'entraînement (3) que le premier mécanisme de guidage (8) ;
dans lequel le premier élément de traction (4) et le deuxième élément de traction (5), enroulés autour du premier arbre d'entraînement (1) dans des directions opposées et partiellement raccordés de manière fixe au premier arbre d'entraînement (1), sont raccordés de manière fixe à l'organe terminal d'instrument (20) après des changements de direction en passant sur un même côté du premier mécanisme de guidage (8) ; et le troisième élément de traction (6) et le quatrième élément de traction (7), enroulés autour du troisième arbre d'entraînement (3) dans des directions opposées et partiellement raccordés de manière fixe au troisième arbre d'entraînement (3), sont raccordés de manière fixe à l'organe terminal d'instrument (20) après des changements de direction en passant sur un même côté du deuxième mécanisme de guidage (9).

3. Mécanisme de transmission selon la revendication 2, comprenant en outre :
des éléments de raccordement (10, 11, 18, 19), comprenant un premier élément de raccordement (10) et un deuxième élément de raccordement (11) ;
dans lequel le premier élément de raccordement (10) et le deuxième élément de raccordement (11) ne s'intersectent pas, et sont chacun perpendiculaires à l'axe du deuxième arbre d'entraînement (2) ; une extrémité du premier élément de raccordement (10) est raccordée de manière fixe au deuxième arbre d'entraînement (2), l'autre extrémité du premier élément de raccordement (10) étant raccordée de manière fixe au premier mécanisme de guidage (8) ; et une extrémité du deuxième élément de raccordement (11) est raccordée de manière fixe au deuxième arbre d'entraînement (2), l'autre extrémité du deuxième élément de raccordement (11) étant raccordée de manière fixe au deuxième mécanisme de guidage (9).

4. Mécanisme de transmission selon la revendication 2, comprenant en outre :
un mécanisme de guidage de sortie (12, 13), comprenant un premier mécanisme de guidage de sortie (12) et un second mécanisme de guidage de sortie (13) ;
dans lequel le mécanisme de guidage de sortie (12, 13) est situé dans un second espace défini par un troisième plan formé par des axes du premier arbre d'entraînement (1) et du troisième arbre d'entraînement (3), un quatrième plan formé par des axes du deuxième arbre d'entraînement (2) et du premier mécanisme de guidage (8), et un cinquième plan formé par des axes du deuxième arbre d'entraînement (2) et du deuxième mécanisme de guidage (9) ; les axes du premier mécanisme de guidage de sortie (12) et du second mécanisme de guidage de sortie (13) sont perpendiculaires à l'axe du deuxième arbre d'entraînement (2), et les axes du premier mécanisme de guidage de sortie (12) et du second mécanisme de guidage de sortie (13) ne s'intersectent pas ;
dans lequel le premier élément de traction (4) et le deuxième élément de traction (5), après des changements de direction en passant sur un même côté du premier mécanisme de guidage (8), sont raccordés de manière fixe à l'organe terminal d'instrument (20) en passant en outre par le premier mécanisme de guidage de sortie (12) ; et le troisième élément de traction (6) et le quatrième élément de traction (7), après des changements de direction en passant sur le même côté du deuxième mécanisme de guidage (9), sont raccordés de manière fixe à l'organe terminal d'instrument (20) en passant en outre par le second mécanisme de guidage de sortie (13).

5. Mécanisme de transmission selon la revendication 4, dans lequel le mécanisme de guidage (8, 9, 16, 17) comprend en outre un troisième mécanisme de guidage (16) et un quatrième mécanisme de guidage (17) qui sont adaptés autour du deuxième arbre d'entraînement (2) ; et le troisième mécanisme de guidage (16) ne coïncide pas avec le quatrième mécanisme de guidage (17) dans une direction axiale du deuxième arbre d'entraînement (2) ;
le premier élément de traction (4) et le deuxième élément de traction (5), après avoir été enroulés autour du premier arbre d'entraînement (1) et des changements de direction en passant sur un côté à la fois du premier mécanisme de guidage (8) et du troisième mécanisme de guidage (16), sont raccordés de manière fixe à l'organe terminal d'instrument (20) en passant en outre par le premier mécanisme de guidage de sortie (12) ; et le troisième élément de traction (6) et le quatrième élément de traction (7), après avoir été enroulés autour du troisième arbre d'entraînement (3) et des changements de direction en passant sur un côté à la fois du deuxième mécanisme de guidage (9) et du quatrième mécanisme de guidage (17), sont raccordés de manière fixe à l'organe terminal d'instrument (20) en passant en outre par le second mécanisme de guidage de sortie (13).

6. Mécanisme de transmission selon la revendication 4, dans lequel le mécanisme de guidage comprend en outre un troisième mécanisme (16) et un quatrième mécanisme de guidage (17) ; le quatrième mécanisme de guidage (17) se trouve plus loin du premier arbre d'entraînement (1) que le troisième mécanisme de guidage (16), et le troisième mécanisme de guidage (16) se trouve plus loin du troisième arbre d'entraînement (3) que le quatrième mécanisme de guidage (17) ; dans lequel le premier élément de traction (4) et le deuxième élément de traction (5), après avoir été enroulés autour du premier arbre d'entraînement (1) et des changements de direction en passant sur un côté à la fois du premier mécanisme de guidage (8) et du troisième mécanisme de guidage (16), sont raccordés de manière fixe à l'organe terminal d'instrument (20) en passant en outre par le premier mécanisme de guidage de sortie (12) ; et le troisième élément de traction (6) et le quatrième élément de traction (7), après avoir été enroulés autour du troisième arbre d'entraînement (3) et des changements de direction en passant sur un côté à la fois du deuxième mécanisme de guidage (9) et du quatrième mécanisme de guidage (17), sont raccordés de manière fixe à l'organe terminal d'instrument (20) en passant en outre par le second mécanisme de guidage de sortie (13).

7. Mécanisme de transmission selon la revendication 6, comprenant en outre : des éléments de raccordement (10, 11, 18, 19), comprenant un troisième élément de raccordement (18) et un quatrième élément de raccordement (19), dans lequel le troisième élément de raccordement (18) et le quatrième élément de raccordement (19) ne s'intersectent pas, et sont chacun perpendiculaires à l'axe du deuxième arbre d'entraînement (2) ; une extrémité du troisième élément de raccordement (18) est raccordée de manière fixe au deuxième arbre d'entraînement (2), l'autre extrémité du troisième élément de raccordement (18) étant raccordée de manière fixe au troisième mécanisme de guidage (16) ; et une extrémité du quatrième élément de raccordement (19) est raccordée de manière fixe au deuxième arbre d'entraînement (2), l'autre extrémité du quatrième élément de raccordement (19) étant raccordée de manière fixe au quatrième mécanisme de guidage (17).

8. Mécanisme de transmission selon la revendication 5 ou 6, dans lequel le premier élément de traction (4) et le deuxième élément de traction (5) passent sur le même côté du premier mécanisme de guidage (8) et du troisième mécanisme de guidage (16), et le troisième élément de traction (6) et le quatrième élément de traction (7) passent sur le même côté du deuxième mécanisme de guidage (9) et du quatrième mécanisme de guidage (17).

9. Mécanisme de transmission selon l'une quelconque des revendications 1 à 7, dans lequel une distance entre l'axe du deuxième arbre d'entraînement (2) et l'axe du premier arbre d'entraînement (1) est égale à une distance entre l'axe du deuxième arbre d'entraînement (2) et l'axe du troisième arbre d'entraînement (3).

10. Mécanisme de transmission selon l'une quelconque des revendications 2 à 7, dans lequel le premier élément de traction (4) et le deuxième élément de traction (5) passent par le premier mécanisme de guidage (8) sur un côté du premier mécanisme de guidage (8) éloigné du deuxième arbre d'entraînement (2), et le troisième élément de traction (6) et le quatrième élément de traction (7) passent par le deuxième mécanisme de guidage (9) sur un côté du deuxième mécanisme de guidage (9) éloigné du deuxième arbre d'entraînement (2).

11. Mécanisme de transmission selon l'une quelconque des revendications 4 à 7, dans lequel le mécanisme de guidage de sortie est situé sur un plan médian vertical du troisième plan, ou le premier mécanisme de guidage de sortie (12) et le second mécanisme de guidage de sortie (13) sont disposés de manière symétrique par rapport au plan médian vertical.

12. Mécanisme de transmission selon l'une quelconque des revendications 4 à 7, dans lequel le premier élément de traction (4) comprend une première extrémité, un premier corps (41) s'étendant à partir de la première extrémité, et une deuxième extrémité raccordée au premier corps (41) ; la première extrémité est enroulée autour du premier arbre d'entraînement (1) et est partiellement raccordée de manière fixe au premier arbre d'entraînement (1) ; le premier corps (41) passe sur un côté du mécanisme de guidage (8, 9), et s'étend jusqu'à la deuxième extrémité après un changement de sa direction par le premier mécanisme de guidage de sortie (12) ; et la deuxième extrémité est raccordée de manière fixe à l'organe terminal d'instrument (20) ;
le deuxième élément de traction (5) comprend une troisième extrémité, un deuxième corps (51) s'étendant à partir de la troisième extrémité, et une quatrième extrémité raccordée au deuxième corps (51) ; la troisième extrémité est enroulée autour du premier arbre d'entraînement (1) dans une direction opposée à la première extrémité, et est partiellement raccordée de manière fixe au premier arbre d'entraînement (1) ; le deuxième corps (51) passe sur un côté du mécanisme de guidage (8, 9), et s'étend jusqu'à la quatrième extrémité après un changement de sa direction par le premier mécanisme de guidage de sortie (12) ; et la quatrième extrémité est raccordée de manière fixe à l'organe terminal d'instrument (20).

13. Mécanisme de transmission selon l'une quelconque des revendications 4 à 7, dans lequel le troisième élément de traction (6) comprend une cinquième extrémité, un troisième corps (61) s'étendant à partir de la cinquième extrémité, et une sixième extrémité raccordée au troisième corps (61) ; la cinquième extrémité est enroulée autour du troisième arbre d'entraînement (3) et est partiellement raccordée de manière fixe au troisième arbre d'entraînement (3) ; le troisième corps (61) passe sur un côté du mécanisme de guidage (8, 9), et s'étend jusqu'à la sixième extrémité après un changement de sa direction par le second mécanisme de guidage de sortie (13) ; et la sixième extrémité est raccordée de manière fixe à l'organe terminal d'instrument (20) ;
le quatrième élément de traction (7) comprend une septième extrémité, un quatrième corps (71) s'étendant à partir de la septième extrémité, et une huitième extrémité raccordée au quatrième corps (71) ; la septième extrémité est enroulée autour du troisième arbre d'entraînement (3) dans une direction opposée à la cinquième extrémité, et est partiellement raccordée de manière fixe au troisième arbre d'entraînement (3) ; le quatrième corps (71) passe sur un côté du mécanisme de guidage (8, 9), et s'étend jusqu'à la huitième extrémité après un changement de sa direction par le second mécanisme de guidage de sortie (13) ; la huitième extrémité est raccordée de manière fixe à l'organe terminal d'instrument (20) ; et le troisième corps (61) et le quatrième corps (71) sont toujours situés sur un même côté du premier corps (41) et du deuxième corps (51).

14. Mécanisme de transmission selon l'une quelconque des revendications 1 à 7, dans lequel le premier arbre d'entraînement (1) et/ou le troisième arbre d'entraînement (3) comprennent en outre un élément de rotation (14, 15) ; l'élément de rotation (14, 15) est raccordé de manière fixe et de manière coaxiale à l'arbre d'entraînement (1, 2, 3) correspondant; l'élément de rotation (14, 15) est pourvu d'un orifice ; et l'élément de traction (4, 5, 6, 7) est enroulé autour de l'élément de rotation (14, 15) de l'arbre d'entraînement (1, 2, 3) correspondant et raccordé de manière fixe à l'orifice de l'élément de rotation (14, 15).

15. Instrument chirurgical, comprenant un organe terminal d'instrument (20) situé à une extrémité de l'instrument chirurgical et le mécanisme de transmission selon l'une quelconque des revendications 1 à 14, dans lequel le mécanisme de transmission est conçu pour amener l'organe terminal d'instrument (20) à effectuer des mouvements.
